# EUROPEAN PATENT APPLICATION

(11) **EP 2 008 682 A2**
(43) Date of publication of application: **31.12.2008**
(21) Application number: 08010820.2
(22) Date of filing: 13.06.2008
(51) Int. Cl.: A61M 25/09

(54) **Medical device**

(30) Priority: 15.06.2007 US 934755 P
(71) Applicant: Olympus Medical Systems Corp., Tokyo 151-0072 (JP)
(72) Inventor: Motai, Kousuke c/o Olympus Med. Sys. Corp., Tokyo 151-0072 (JP); Okada, Tsutomu c/o Olympus Med. Sys. Corp., Tokyo 151-0072 (JP); Miyamoto, Satoshi c/o Olympus Med. Sys. Corp., Tokyo 151-0072 (JP)
(74) Representative: von Hellfeld, Axel

(57) **Abstract**

A medical device which is used by inserting a body cavity comprising a wire at least a part thereof has flexibility, and a tip which is attached to a distal end of the wire. A central axis of the tip is displaced from a central axis of the wire

## Description

### BACKGROUND OF THE INVENTION

### Field of the Invention

The present invention relates to a medical device to be inserted through natural orifices.

### Background Art

After inserting a catheter into a bile duct or a vaginal canal, when getting through flexure or narrow parts with a guide wire, the guide wire is manipulated by a combined operation of pushing or pulling and rotating while verifying the tip location of the guide wire through a two-dimensional X-Ray image so as to get through the flexure and narrow parts.

### SUMMARY OF THE INVENTION

The present invention provides a medical device which is used by inserting a body cavity; the device comprising a wire at least a part thereof has flexibility, and a tip which is attached to a distal end of the wire; wherein a central axis of the tip is displaced from a central axis of the wire.

### BRIEF DESCRIPTION OF THE DRAWINGS

FIG. 1 is a diagram showing a flexure/narrow part-penetrating device which is a first embodiment of a medical device of the present invention.
FIG. 2 is a diagram showing a performance of the flexure/narrow part-penetrating device in use.
FIG 3 is a diagram showing the performance of the flexure/narrow part-penetrating device in use.
FIG. 4 is a diagram showing a performance of a conventional flexure/narrow part-penetrating device in use.
FIG. 5 is a diagram showing a flexure/narrow part-penetrating device which is a second embodiment of the present invention.
FIG. 6 is a diagram showing a performance of the flexure/narrow part-penetrating device in use.
FIG 7 is a diagram showing the performance of the flexure/narrow part-penetrating device in use.
FIG. 8 is a diagram showing the performance of the flexure/narrow part-penetrating device when the device is drawn into a cannula.
FIG. 9 is a diagram showing a flexure/narrow part-penetrating device which is a third embodiment of the present invention.
FIG. 10 is a diagram showing a shape of a bile duct.
FIG 11 is an enlarged diagram showing a distal end of the flexure/narrow part-penetrating device.
FIG 12 is a diagram showing a performance of the flexure/narrow part-penetrating device which is a fourth embodiment of the present invention in use.
FIG. 13 is a diagram showing a performance of a conventional wire in use.
FIG. 14 is a diagram showing a flexure/narrow part-penetrating device which is a fifth embodiment of the present invention.
FIG. 15 is a diagram showing a performance of the flexure/narrow part-penetrating device in use.
FIG 16 is a diagram showing the performance of the flexure/narrow part-penetrating device in use.
FIG 17 is a diagram showing the performance of the flexure/narrow part-penetrating device in use.

### PREERRRED EMBODIMENTS

Embodiments of the invention will be illustrated. Hereinafter, the same reference numerals are given to similar constitutional elements of each embodiment. In addition, overlapping descriptions will be omitted.

### [First Embodiment]

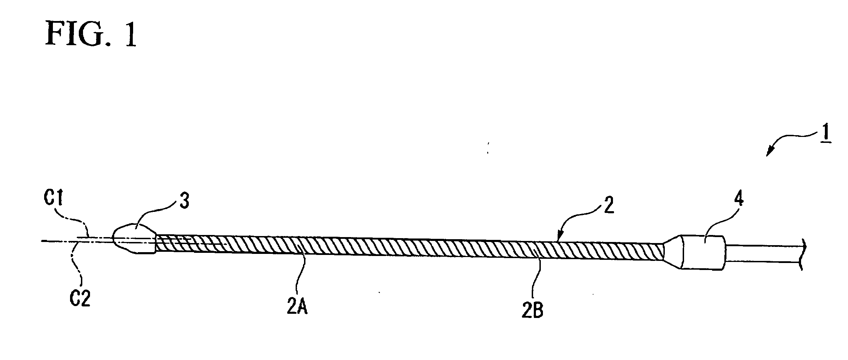

As shown in FIG. 1, with regard to a flexure/narrow part-penetrating device 1 (hereinafter called as "device") which is a medical device, a distal tip 3 is attached to the distal end of a wire 2 that is long, flexible, and excellent in torque transmissibility. The wire so called "torque wire" can appropriately be employed as the wire 2.

The wire 2 includes the first portion 2A and the second portion 2B, which differ in hardness. To the first portion 2A in the distal side, the distal tip 3 is attached. The second portion 2B continues from the proximal end of the first portion 2A, and is harder than the first portion 2A. Furthermore, a diameter of the second portion 2B is larger than that of the first portion 2A. To the proximal end of the second 2B, an operating portion is attached to be grabbed by a technician for manipulation. The operating portion 4 is detachable from the wire 2.

A central axis C1 of the distal tip 3 is displaced from a central axis C2 of the wire 2. The outer diameter of the distal tip 3 is larger than that of the first portion 2A of the wire 2. The distal tip 3 is harder than the first portion 2A of the wire 2.

This device 1 is inserted through a patient's natural orifice, for example, a mouth, and can be easily passed through a flexure or narrow part of a bile duct or a vaginal canal.

A performance of the device 1 in use in the flexure of the bile duct will be illustrated as an example. First of all, a catheter (not shown in figure) is inserted through a mouth to just in front of a flexure of a bile duct. The device 1 is inserted into the catheter. The device 1 is inserted into the bile duct along the catheter.

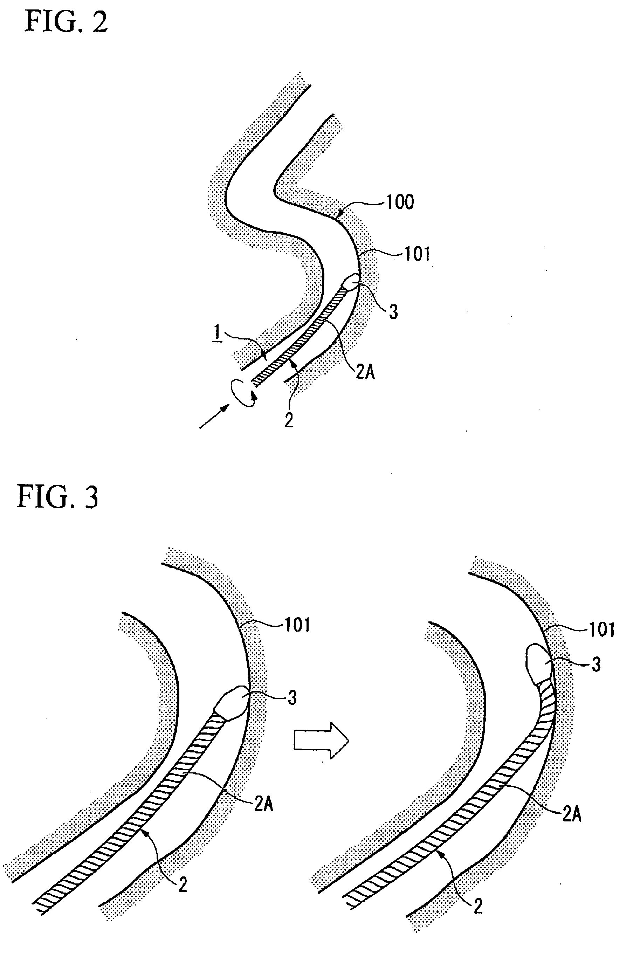

As shown in FIG. 2, the device 1 is rotated while being pushed in the flexure 101 of the bile duct 100 as pointed with arrows. The distal tip 3 is rotated via the wire 2 which has excellent torque transmission. As shown in FIG. 3, it is easy for the distal tip 3 to turn toward the direction capable of passing through the flexure 101 as the distal tip 3 is eccentric to the wire 2. In addition, it is easy for the distal tip 3 to change direction because the diameter of the distal tip 3 is larger than that of the wire 2.

While bending the first part 2A of the wire 2 so as to trace the distal tip 3 in which the direction thereof has been changed, the device 1 is pushed along the flexure 101 of the bile duct 100.

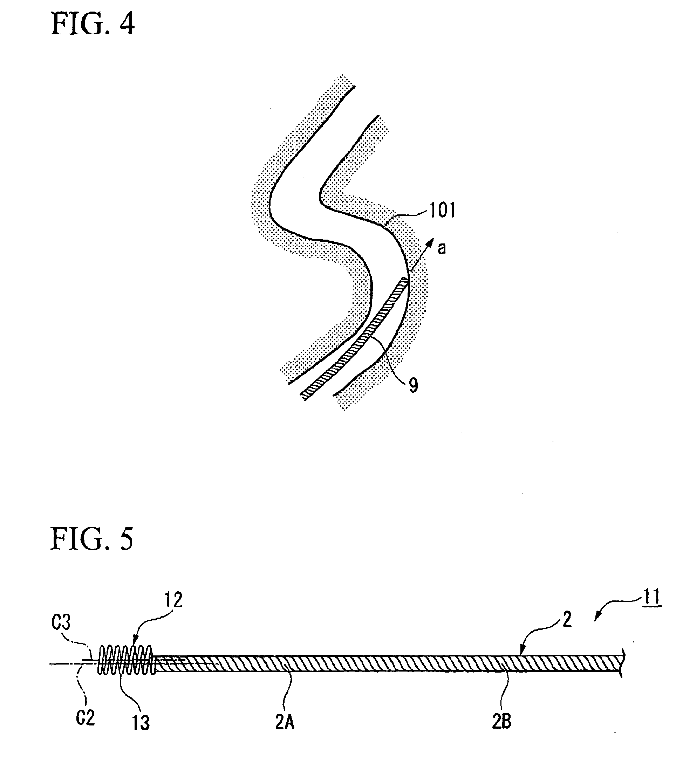

As shown in FIG 4, it had been difficult to pass through the flexure 101 with a conventional device 9 because a force is only applied to a direction pointed with an arrow 'a'. The device 1 of the present embodiment enables easy passage through the flexure 101 by rotating the wire 2 while pushing.

Here, while the device 1 is used, it is also possible not to employ a catheter.

### [Second Embodiment]

As shown in FIG. 5, in a device 11 which is a medical device according to this embodiment, a coil 13 is attached as a distal tip 12 thereof. A central axis C1 of the distal tip 12 (which is approximately same as a central axis of a loop of the coil 13) is displaced from the central axis C2 of the wire 2. An outer diameter of the distal tip 12 is larger than that of the wire.

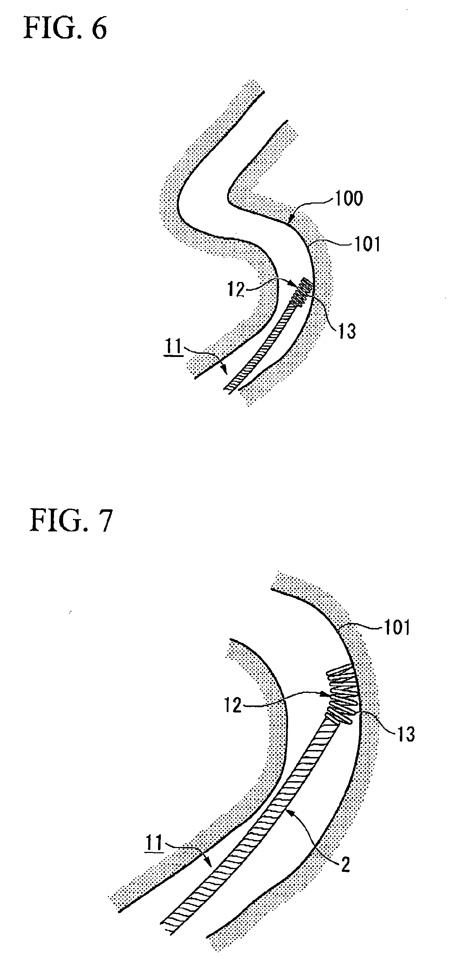

As shown in FIG. 6, when passing through the flexure 101 by operating the device 11, the wire 2 is inserted into the bile duct 100 until the coil 13 which act as the distal tip 12 reaches an inner wall thereof. Furthermore, the device 11 is rotated while being pushed, and the coil 13 which hitting against the inner wall of the bile duct 100 is compressed by being pushed. The coil 13 is turned toward a direction capable of passing through the flexure 101 as pointed with an arrow in FIG. 7 by rotating the device 11, and then the coil 13 can be passed through the flexure 101 due to the restoring force thereof.

With regard to the device 11, since the coil 13 is employed as the distal tip 12, it facilitates passing through the flexure 101 by being rotated while being pushed.

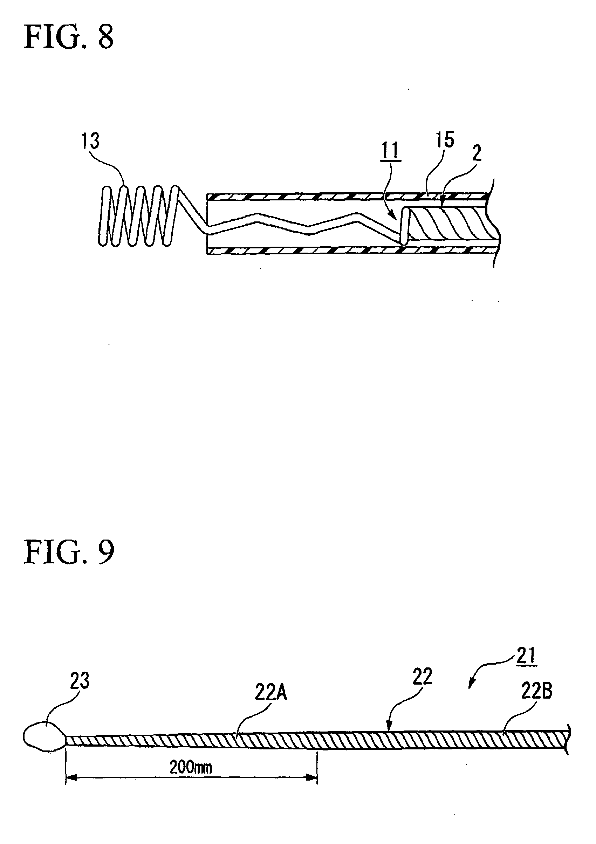

In addition, as shown in FIG. 8, when the wire 2 is drawn into a cannula 15, the loop of the coil 13 deforms to a linear shape and the coil 13 can be inserted into the cannula 15.

In the conventional flexure/narrow part-penetrating device, in case of the insertion of the device using an inner cavity of the cannula after the insertion of the cannula into the bile duct, the outer diameter of the device have to be smaller than the inner diameter of the cannula. However, in case of the device 11, the coil13 can be inserted into the cannula 15 by deforming the loop of the coil 13 to the linear shape. Therefore, the insertion of the device can be performed by using the cannula even though the device which has outer diameter larger than the inner diameter of the cannula is provided.

In general, the more the diameter of the distal tip is larger than that of the proximal wire, and the more the distal tip is harder than the proximal member, the distal end of the flexure/narrow part-penetrating device is easily be bent. Therefore, by employing the structure according to the device 11, the device which enables fit to the flexure more easily and has high capacity for insertion.

Furthermore, in place of the distal tip which is formed by the coil 13, the distal tip having a diameter larger than the cannula 15 may be formed using a shape memory alloy and the like. In this case, the distal tip which can deform the shape enables to stow in the cannula 15 such as the linear shape depend on the predetermined temperature condition can be provided.

The central axis C1 of the wire 2 having excellent torque transmissibility may be brought in line with a central axis C2 of a proximal coil 13. The device can be easily passed through the flexure by taking advantage of a restoring force of the coil 13.

### [Third Embodiment]

As shown in FIG. 9, in a device 21 of this embodiment, the diameter of a distal portion of a wire 22 is reduced in a tapered form to form a first portion 22A. In the proximal side thereof, a second portion 22B is formed identical to the above-described device 1.

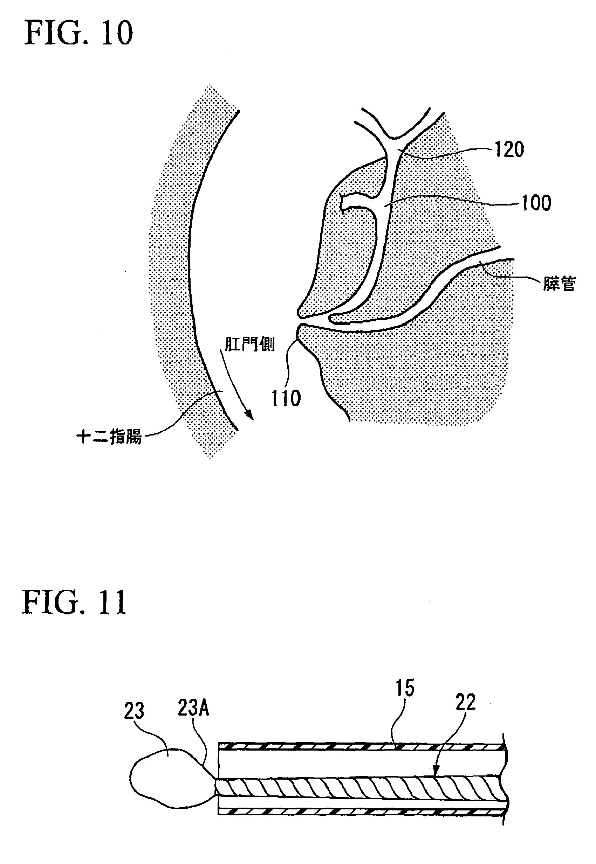

The length of the first portion 22A is approximately 200 mm, and the more distal, the smaller the diameter is. It is known that the length of the bile duct 100 from papilla110 to the hepatic portal region 120 shown in FIG. 10 is anatomically about 200 mm even though there is individual difference. Accordingly, since 200 mm of the distal portion of the wire 2 is in a taper form, the device can approach the hepatic portal region 120 with a tapered flexible first portion 22A, and thus facilitate entry into a deep area of the bile duct 100.

Moreover, the second portion 22B which is not tapered is harder than that of the first portion 22A which is tapered, and thus the second portion 22B is excellent in torque transmissibility. Since the first portion 22A is soft, its shape is easily deformed in accordance with an inflective lumen.

As shown in FIG. 11, a distal tip 23 is tapered so as to gradually reduce the diameter from the distal part to the proximal part which is connected to the wire 22. Owing to the tapered part 23A, it is difficult for the distal tip 23 to get stuck when drawn into the cannula 15.

### [Fourth Embodiment]

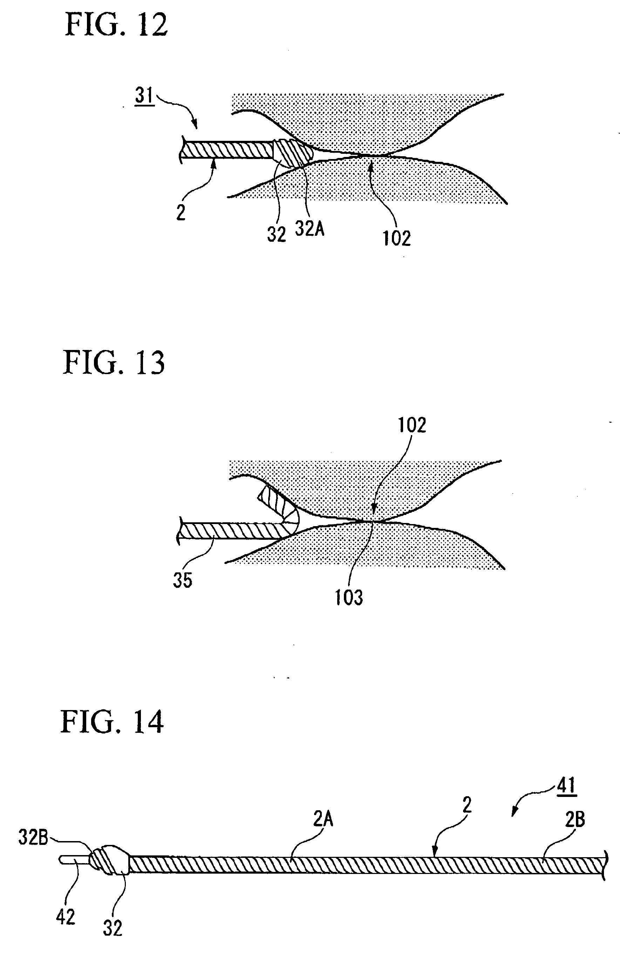

As shown in FIG. 12, in a device 31 of this embodiment, a spiral groove 32A is notched on a distal tip 32, and then the device is spindle-shaped as a whole.

When passing through a narrow part 102 by operating the device 31, the device 31 is inserted through a natural orifice and is advanced in a body cavity until the distal tip 32 hits the narrow part 102 as shown in FIG..12.

When the device 31 is rotated, the narrow part 102 engages with the spiral groove 32A of the distal tip 32 thereby creating a propulsion force, which enables passage through the narrow part 102.

As shown in FIG. 13, it had been difficult to get through the narrow part with a conventional guide wire 35 although it is used to get through the narrow part, because the guide wire 35 would be folded so as to make a loop in front of the narrow part 102 if the axis of the guide wire 35 does not match a space 103 capable of passing into a narrow part 102.

According to the device 31 of this embodiment, since the distal tip 32 has the spiral groove 32A and then the device is spindle-shaped, the device can be easily passed through a narrow part by being rotated while being pushed.

In this embodiment, the distal tip 32 and the wire may be provided around a same axis or they may be provided around different axes.

### [Fifth Embodiment]

As shown in FIG. 14, with regard to a flexure/narrow part-penetrating device 41 which is a medical device, a flexible wire 42 (second wire) is stuck out from the apex of a distal tip 32, in addition to that a spiral groove 32B is provided on the circumference of the distal tip 32 which is attached to a wire 2. The wire 42 may be formed by projecting the part of the first portion 1A of the wire 2, or may be formed by attaching other member onto the wire 2.

Since the spiral groove 32B is provided on a part of the distal tip 32, particularly only on a distal part thereof, the spiral groove does not resist pulling out the wire 2. That is, the spiral groove does not hinder pulling out the wire 2.

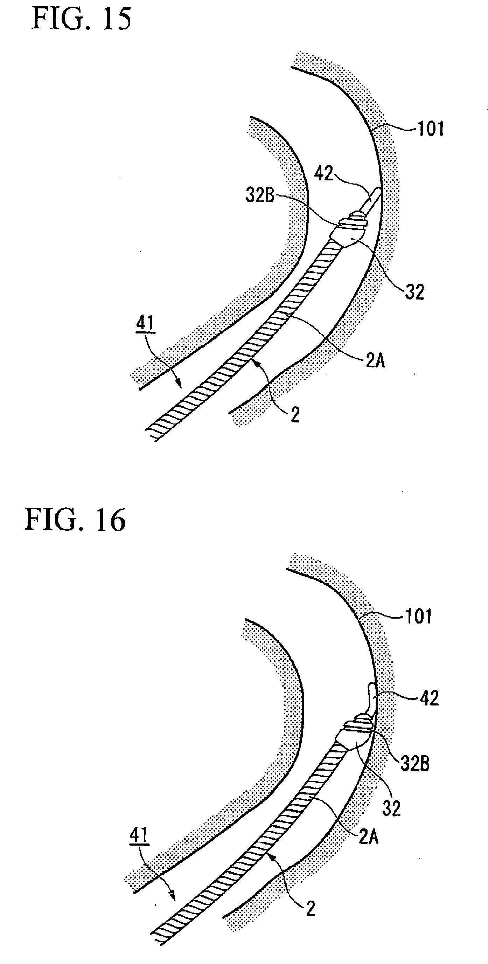

When the device 41 is inserted into a flexure 101 as shown in FIG. 15, the flexible wire 42 hits a wall of the flexure because the flexible wire 42 is stuck out from the distal end of the distal tip 32. At this time, the flexible wire 42 deforms as shown in FIG. 16 by adding more force. The flexible wire 42 tends to turn toward the direction for passing a flexure, and thus facilitates passage through a flexure.

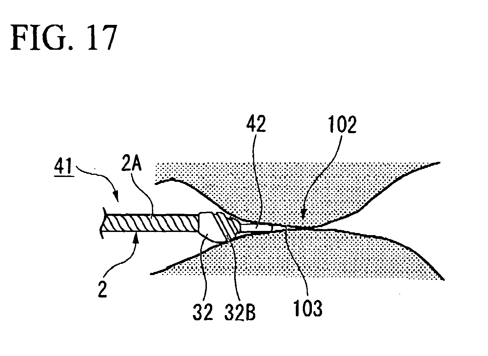

Furthermore, as shown in FIG 17, in the narrow part 102, the flexible wire 42 gets stuck in a space 103 in which the flexible wire can be passed in the narrow part 102, and thus an opportunity for the distal tip 32 to screw in the narrow part using the spiral groove 32B can be provided. Consequently, it enables to simple passage through narrow parts.

In this embodiment, the distal tip 32 is provided with the flexible wire 42 and the spiral groove 32B, and therefore allows the simple passage through narrow parts or flexures by rotating the wire while it is being pushed.

Furthermore, these embodiments may be suitably combined.

## Claims

1. A medical device which is used by inserting a body cavity, comprising:
a wire at least a part thereof has flexibility, and
a tip which is attached to a distal end of said wire;
wherein a central axis of said tip is displaced from a central axis of said wire.

2. A medical device according to claim 1, wherein said wire includes a first portion which is provided at a distal side thereof and has flexibility, and a second portion, which is provided at a proximal side thereof and is harder than said first portion.

3. A medical device according to claim 2, wherein a torque wire is employed as said second portion of said wire.

4. A medical device according to claim 1, wherein a diameter of said tip is larger than that of said wire.

5. A medical device according to claim 1, wherein a spiral groove is provided on a circumference of said tip.

6. A medical device according to claim 5, wherein said groove is only provided on a distal side of said tip.

7. A medical device according to claim 1, wherein a part of said tip which includes a proximal end connected to the wire is tapered so as to gradually reduce the diameter to the proximal end.

8. A medical device which is used by inserting a body cavity, comprising:
a first wire at least a part thereof has flexibility,
a tip which is attached to a distal end of said first wire, and
a second wire at least a part thereof has flexibility and is attached to a distal end of said tip.

9. A medical device which is used by inserting a body cavity, comprising:
a first wire at least a part thereof has flexibility, and
a coil which is attached to a distal end of said first wire.

10. A medical device according to claim 9, wherein a diameter of a loop of said coil is larger than a diameter of said wire.

11. A medical device according to claim 9, wherein said coil deforms to a linear shape by loosening a loop due to add a force greater than the predetermined value along an axial direction thereof, and said coil can be drawn into a cannula having a diameter smaller than that of said loop.
